# EUROPEAN PATENT SPECIFICATION

(11) **EP 0 567 423 B1**
(45) Date of publication and mention of the grant of the patent: **05.02.1997**
(21) Application number: 93500050.5
(22) Date of filing: 23.04.1993
(51) Int. Cl.: A61B 17/58

(54) **Transpedicular vertebral attachment systems and device for the practice thereof**
Transredikuläres Wirbelkörperbefestigungssystem und Vorrichtung zur Ausführung davon
Systèmes transpediculaires de fixation de vertèbres et dispositif pour les mettre en pratique

(30) Priority: 24.04.1992 ES 9200870; 01.08.1992 ES 9201628; 05.02.1993 ES 9300215
(43) Date of publication of application: 27.10.1993
(73) Proprietor: Barbera Alacreu, José Vicente, E-46009 Valencia (ES)
(72) Inventor: Barbera Alacreu, José Vicente, E-46009 Valencia (ES)
(74) Representative: Blumbach, Kramer & Partner

(56) References cited:
- EP-A- 0 340 159
- EP-A- 0 384 001
- FR-A- 2 615 095
- US-A- 4 620 533

## Description

The present invention relates to transpedicular vertebral attachment systems providing for their intended function various advantages to be described hereinafter, apart from others inherent in the organization and constitution thereof.

There is currently known the technique regularly used for transpedicular vertebral attachment using a normal five centimeter long screw.

In addition, there are known from FR-A 2,615,095 transpedicular vertebral attachment systems wherein two long screws are used which are applied from the back side.

This technique suffers from drawbacks derived from the very dimension and constitution of the screw and of the anchorage site, which may cause rocking of the front end of the screw, proper to osteoporotic vertebrae, with the consequent loss of pull-out strength and with the serious risk of extending beyond the vertebra anterior cortical layer with the tip of the screw, possibly perforating viscera or blood vessels.

In addition to these systems, the document EP-A 0 340 159 discloses a transpedicular vertebral attachment system comprising a plug member having a bore and having a plurality of longitudinally extending slits which are expanded radially when a screw member is inserted into the bore of the plug member. Said plug member is provided with circumferential grooves. A disadvantage of such grooves is that the hole drilled into the bone before inserting the plug member is widened when the plug member is inserted before an expansion of the plug member is accomplished. By widening the hole, additional bone material is removed resulting in a less tight fit of the plug member in the hole even when the plug member is expanded by the insertion of the screw member.

The present inventors have devised improvements in transpedicular vertebral attachment systems of the type described above, consisting of replacing the traditional length screws with another much shorter screw applied on a plug of silastic or any other polymer of similar mechanical features and biocompatibility, or any metal or alloy, which is inserted in the pedicle and being only slightly longer than the latter.

Document EP-B 0 220 736 discloses an apparatus for the fixation of a vertebra comprising a deformable rod maintaining at least three vertebrae in a fixed relationship, clamp means for gripping said rod and fastener means for securing said clamp means to a vertebra. Said fastener means is provided with threaded portions which allow the threading into a hole in the vertebra in the one side and the engagement of a nut on the other side in order to press and secure the clamp (and with the clamp the rod) abuttingly to the vertebra.

Document EP-B 0 325 682 is directed to a bone screw having two threaded portions and one unthreaded portion, the latter one being located intermediate the threaded portions and having the function to restrict movement of the screw in traverse direction relative to the axis of the screw and to prevent effluence from the opening in the bone portion which the one of the threaded portions is screwed into.

Document EP-A 0 567 423 (prior art by virtue of Art. 54 (3) EPC) is concerned with a spinal column retaining apparatus comprising a fastener means for engaging the vertebra, a rod member which affords stability of the spinal column after application of the retaining apparatus and a connector means which enables the rod member to be positioned about an axis spaced from the fastener means and extending at an angle preferably perpendicular to the axis of the rod member and further the distance between the rod member and the fastener means to be varied.

Document EP-A 0 534 053 (prior art by virtue of Art. 54 (3) EPC) discloses a vertebral locking and retrieving system by which two neighbouring vertebrae can be interconnected and stabilized. A locking pin or fastener and a remedial screw each of which are screwed into the respective vertebrae to be stabilized, are interconnected by a threaded rod member via coupling components.

The objective is that when the screw is screwed into the plug, the front inner end of the latter is widened on being expanded by the screw.

The invention concerns a transpedicular vertebral attachment system comprising
- a plug member 2 having a bore and a plurality of longitudinally extending slits 7;
- a screw member 1, 3 inserted into the bore of the plug member 2 which is expanded radially thereby;
characterized in that the plug member 2 comprises a circular cross section and a thread 6 on the outer surface thereof, and means 5 for rotating the plug member 2.

The following purposes are obtained:
1) a shorter screw, avoiding the danger of perforating viscera or blood vessels, which may happen when the conventional screws are used.
2) a greater resistance to pull-out forces, since the inner end of the plug is much wider than the outer end, preventing it from being forced out backwards. Likewise, since an exclusively pedicular anchorage is available, this being the strongest part of the vertebra, the possibility of rocking movements of the front end of the screw, proper to osteoporotic vertebra, is avoided.

Thus, the improvements based on the use of "shorter screw + plug" include the advantages of the other systems, while also providing the improvement derived from the fact of applying the forces exclusively on the most solid part of the vertebra, i.e. the pedicle. It also avoids the risk of the pull-out strength being lost in osteoporotic vertebrae, since the inner portion of the plug is wider than the outer portion. Finally, with the improvements of the invention, it is not possible to exceed the anterior cortical layer of the vertebra with the screw tip, thereby eliminating the concomitant risks.

Thus, the improvements provide greater security and support when this technique is used in osteoporotic patients in which the density of the bone tissue of the vertebral body is much less.

Also forming part of the invention are means devised to complete the intersegmentary attachment of the vertebra, for joining the screws inserted in the vertebral pedicles together.

Such means are formed essentially by rods of special characteristics for attachment between the anchored levels and by gripper arms also specially structured to form clamps for fixation of the said rods to the pedicular screws, with the aid of round headed blind nuts. Hence, in another embodiment, the invention is concerned with a transpedicular vertebral attachment system as described above, which attachment system further comprises
- at least two pairs of clamp members 18, each pair consisting of two single clamp members comprising two holes 20, 21 and a groove 22, the first of said two holes 20 having an inner diameter corresponding to the outer diameter of said plug member 2, 24 and being suitable to house said thread 6 on the outer surface of said plug member 2, 24, the second of said two holes 21 having a smaller diameter than said first hole 20 and being suitable to house a small screw 25 for holding and joining said pair of clamp members 18 tight together, and said groove 22 having the form of a half of a cylinder;
- one pair of round headed blind nuts 26, 27 screwed on each plug member 2, 24 housed in said holes 20 of said pair of clamp members 18, said round headed blind nuts 26, 27 being adapted to hold and join said pair of clamp members 18 tight together; and
- a cylindrical rod 16 having an outer diameter corresponding to the inner diameter of said pair of grooves 22 of said pair of clamp members 18, said rod 16 fitting to and being fixed by said pair of grooves 22.

In an alternative embodiment, the said means provide a semi-open connexion for the anchorage of the rods and the pedicular plug.

In accordance with this embodiment, the invention concerns a transpedicular vertebral attachment system as described above, which attachment system further comprises
- a cylindrical circular clamp member 29 open at one of its sides and housing therein a ball joint 31 which is also open at one side thereof;
- a pair of hollow cylinders 30 one of them each being attached to said open sides of said cylindrical circular clamp member 29, said hollow cylinders being designed to receive said screw member 1, 3, 34 before the latter is inserted into the bore of the plug member 2, 24; and
- a cylindrical rod 32 being designed to be inserted through said circular clamp member 29 and said ball joint 31, said rod 32 fitting to and being fixed by said clamp member 29 and said ball joint 31.

The improvements of the present invention afford the advantages described above, apart from others which will be readily gathered from the embodiment of the vertebral attachment system with exclusive transpedicular support provided with such improvements, described in further detail hereinafter to facilitate the understanding of the above features, while various details are disclosed at the same time. The present description is accompanied by drawings in which there is shown one embodiment of the invention just as an example and without limitation of the scope thereof.

In the drawings:
Figure 1 shows the screw forming a part of the device for carrying out the system.
Figures 2, 3 and 4 are different projections of the plug forming part of the said device.
Figure 5 is a view of the device, comprising the screw and plug.
Figures 6, 7 and 8 show different stages of the system in which the technique for fitting the device is shown.
Figure 9 corresponds to the prior art, showing the risks involved with the use of a conventionally sized pedicular screw.
Figure 10 is an exploded perspective view of the said components, being assembled according to an alternative embodiment consisting of a bar and two gripper arms forming the clamp.
Figure 11 is a perspective view showing the vertebral intersegmentary attachment system, according to Figure 14.
Figure 12 is a sectional view of a gripper arm.
Figure 13 is a sectional view of two assembled gripper arms forming a clamp.
Figure 14 shows the plug and screw passing through the two gripper arms in form of a clamp which in turn hold a connecting bar.
Figure 15 is a plan view from below of a clamp according to an alternative embodiment.
Figure 16 is a sectional view of the clamp, on the line A-A' of Figure 15.
Figure 17 is a similar view to that of Figure 15, in which the clamp includes the screw to be inserted in the pedicular plug.
Figure 18 is a schematic view of the clamp with the fixing bar and the cylindrical member with the screw and pedicular plug.

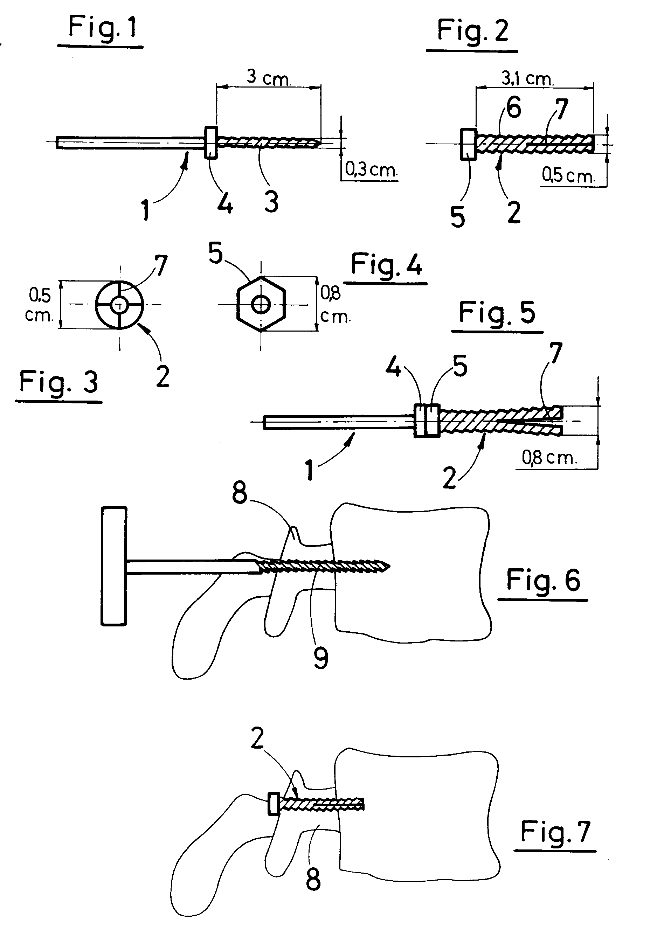

With reference to Figures 1 to 9, there is to be seen a system designed for vertebral attachment exclusively with transpedicular support, comprising a screw member 1 which is preferably of of steel, titanium or any alloy, designated in general by 1, and a plug 2 which is preferably of silastic or any other polymer of similar mechanical properties and biocompatibility, or of any metal or alloy.

The screw member 1 has a threaded portion 3, limited by a radial widened portion 4, acting as a stop limiting the insertion of the screw member 1 into the plug member 2. Preferably, the threaded portion 3 extends between the screw tip and said widened portion 4. In a further preferred embodiment, the threaded portion 3 of the screw member is slightly longer than the length of a pedicle to which the system is to be applied.

The plug 2 has a radial widening 5 to prevent it being inserted too far in, and the shank of said plug is provided with a thread 6. The widened portion 5 preferably is hexagonal in shape to allow it to engage a hollow screwdriver, allowing it to be manipulated.

The said plug 2 is of the type having longitudinally extending slits 7 to promote the expansion thereof.

The process for applying the above system comprises a first step in which the pedicle 8 is drilled, with a twist drill 9, of e.g. 0.4 cm, as shown in Figure 6.

In a second step, as shown in Figure 7, the plug member 2 is inserted in the pedicle 8, preferably by screwing into the bore formed with the twist drill 9.

Finally, in a third step, the threaded portion 3 of the screw 1 is inserted in the plug 2 and screwed into the bore of the said plug.

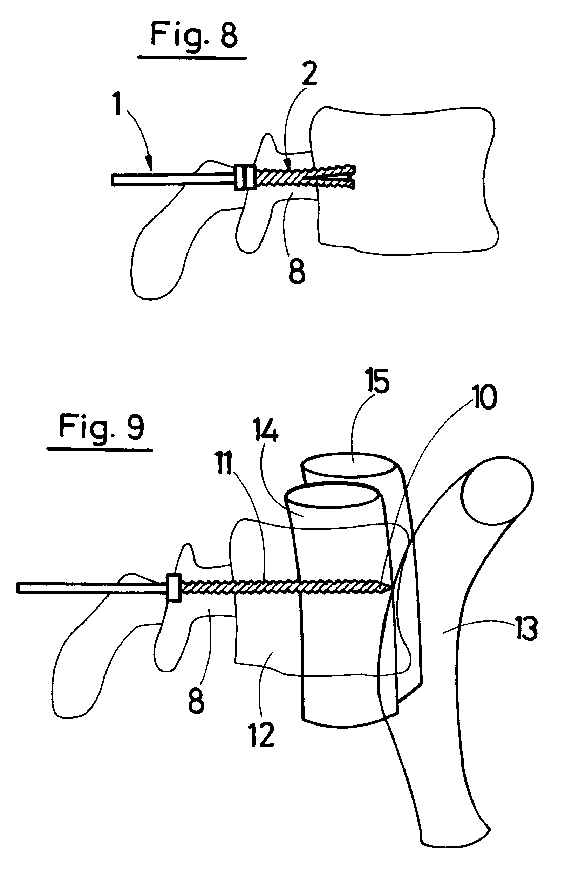

In Figure 8 the advantages provided by the improvements of the invention over the prior art, shown in Figure 9, are clearly to be seen. In Figure 9 showing the risks of the prior art, the tip 10 of the conventional pedicular screw 11 is shown to be in a position to extend beyond the anterior cortical layer of the vertebra 12 and perforate viscera or blood vessels (intestine 13, aorta 14, vena cava 15)

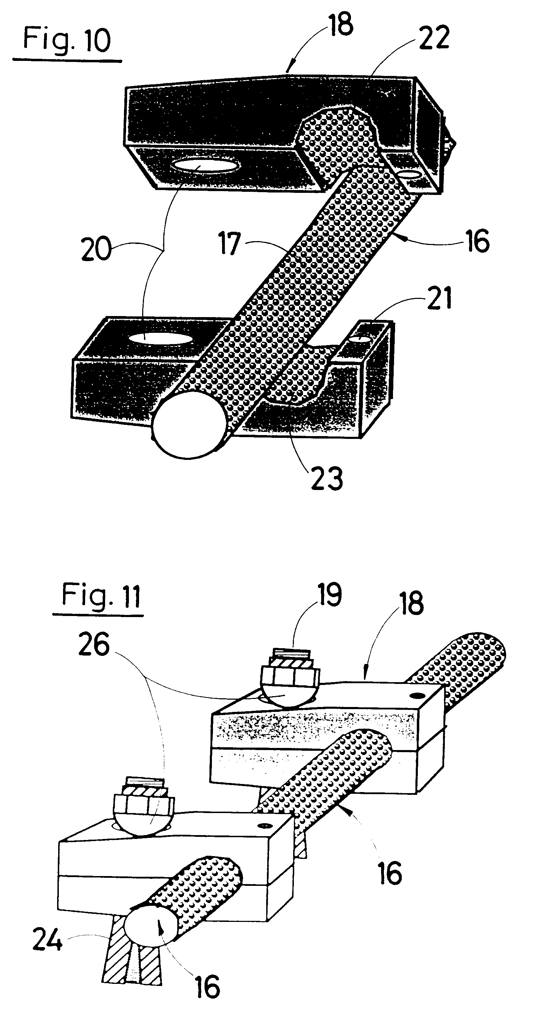

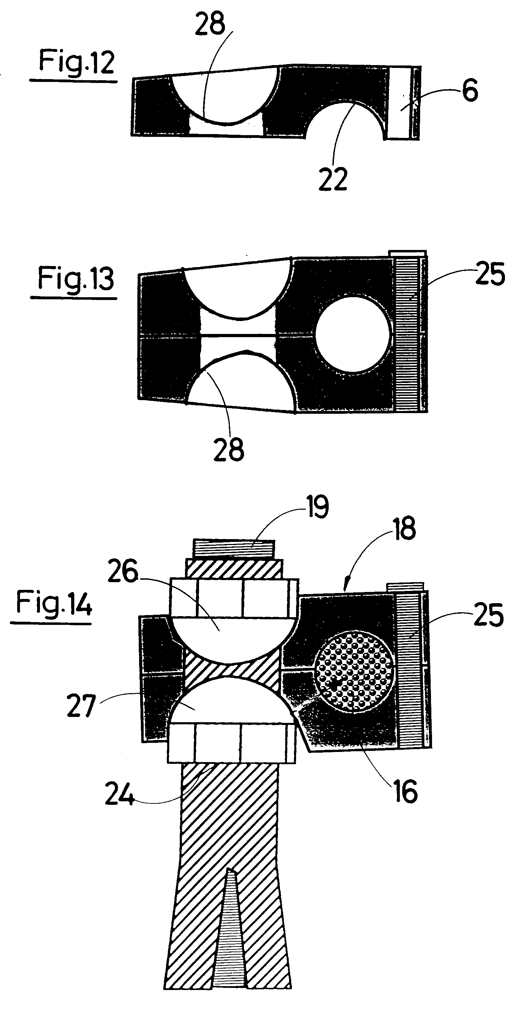

With reference to Figures 10 to 14, there are to be seen rods, designated with 16 formed of the same material as the plug, or of a different, but also biocompatible material, which have a section of e.g. 4.6 and 6 mm and lengths ranging from e.g. 3 to 15 cm. The rods 16 are cylindrical and the surface thereof is preferably finished in 0.5 mm high diamond points 17 to prevent slipping.

The clamps generally designated with 18, are used to attach the rods 16 to the pedicular screws 19. They are formed by two preferably symmetrical members, each of them of rectangular form, having two holes 20 and 21 and a groove 22. The groove runs from top to bottom and the surface preferably is diamond point finished at 23, for holding the rod 16, preventing slippage thereof. The first (larger) hole 20 has the same bore as the section of the pedicular plug 24, which it has to house. The second (smaller) hole 21, located on the opposite side to the larger first one, is for housing a small screw 25 which joins the two gripper arms together, helping firmly to hold the rod 16 between them, preventing slippage thereof.

The attachment of the pedicular plugs 24 to the clamps 18 is consolidated by two round headed blind nuts 26 and 27 screwed on the plug. Said blind nuts 26 and 27 hold the two gripper arms between them, after the plug has been inserted in the corresponding hole 20 in the gripper arms. The thickness of the gripper arms is reduced at this level. The holes for the screw preferably are also spherically hollowed out at 28 to allow any angle of attack of the plug 24 against the gripper arm.

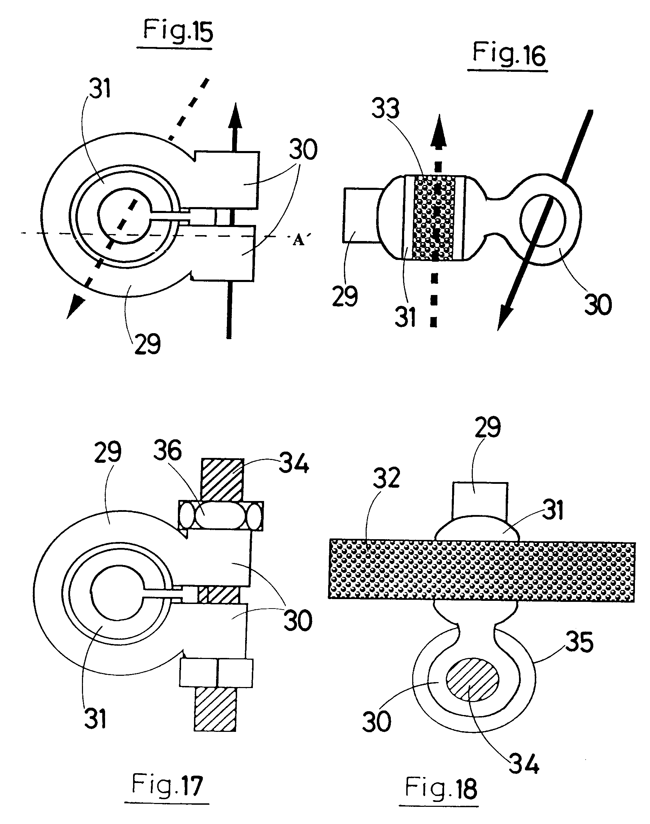

With reference to Figures 15 to 18, there is to be seen an alternative embodiment of clamp connection means applicable in the vertebral fusion systems by way of pedicular plug and screw and connecting rod.

Such connecting means or clamps are constituted by two parts, designated 29 and 30, respectively.

Part 29 is a cylindrical circular clamp member open at one of the sides thereof and housing therein a ball joint 31, also open at one side thereof.

Part 30 constitutes two hollow cylinders, each attached to one of the open ends of the circular member 29.

The attachment rod 32 is inserted through the circular member 29 and the ball joint 31. For the ball joint to grip the rod firmly, the surface internal thereof for engaging the rod 32 is provided with a diamond point finish 33.

A screw 34 is inserted through the two portions of the cylindrical member and is then inserted in the pedicular plug. Once the screw has been inserted, the system is blocked with a nut 36.

In Figures 15 and 16, the dotted arrow shows the passage of the rod and the solid arrow the passage of the pedicular screw.

## Claims

1. A transpedicular vertebral attachment system comprising
- a plug member (2) having a bore and a plurality of longitudinally extending slits (7);
- a screw member (1, 3) inserted into the bore of the plug member (2) which is expanded radially thereby;
**characterized in that** the plug member (2) comprises a circular cross section and a thread (6) on the outer surface thereof, and means (5) for rotating the plug member (2).

2. The transpedicular vertebral attachment system as claimed in claim 1, wherein said means (5) for rotating the plug member (2) has hexagonal outer shape thereby allowing engagement to a hollow screwdriver for manipulation.

3. The transpedicular vertebral attachment system as claimed in claim 1 or in claim 2, wherein said plug member (2) is made of a material selected from the group consisting of silastic, any other polymer of similar mechanical features and biocompatibility, metal and metal alloy.

4. The transpedicular vertebral attachment system as claimed in any of the claims 1 ro 3, wherein the screw member (1, 3) has the form of a cylindrical rod having a threaded portion (3) and a radially widened portion (4) acting as a stop limiting the insertion of the screw member (1, 3) into the plug member (2).

5. The transpedicular vertebral attachment system as claimed in claim 4, wherein said threaded portion (3) of the screw member (1, 3) extends in length between the screw tip and said radially widened portion (4).

6. The transpedicular vertebral attachment system as claimed in claim 5 wherein said threaded portion is slightly longer than the length of a pedicle to be applied to.

7. The transpedicular vertebral attachment system as claimed in any of the claims 1 to 6, wherein the screw member (1, 3) is made of a material selected from the group consisting of steel, titanium or an alloy.

8. The transpedicular vertebral attachment system as claimed in any of the claims 1 to 7, further comprising
- at least two pairs of clamp members (18), each pair consisting of two single clamp members comprising two holes (20, 21) and a groove (22), the first of said two holes (20) having an inner diameter corresponding to the outer diameter of said plug member (2, 24) and being suitable to house said thread (6) on the outer surface of said plug member (2, 24), the second of said two holes (21) having a smaller diameter than said first hole (20) and being suitable to house a small screw (25) for holding and joining said pair of clamp members (18) tight together, and said groove (22) having the form of a half of a cylinder;
- one pair of round headed blind nuts (26, 27) screwed on each plug member (2, 24) housed in said holes (20) of said pair of clamp members (18), said round headed blind nuts (26, 27) being adapted to hold and join said pair of clamp members (18) tight together; and
- a cylindrical rod (16) having an outer diameter corresponding to the inner diameter of said pair of grooves (22) of said pair of clamp members (18), said rod (16) fitting to and being fixed by said pair of grooves (22).

9. The transpedicular vertebral attachment system as claimed in claim 8, wherein said two clamp members (18) of said pair of clamp members (18) are mirror symmetrical with respect to their surfaces to be fixed to each other.

10. The transpedicular vertebral attachment system as claimed in claim 8 or in claim 9, wherein said holes (20) in said clamp members (18) have spherically hollowed-out portions (28) to receive said pair of round headed blind nuts (26, 27) screwed on said plug member (2, 24).

11. The transpedicular vertebral attachment system as claimed in any of the claims 8 to 10, wherein the outer surfaces of said cylindrical rod (16) and/or the inner surfaces (23) of said grooves (22) are diamond-point finished to prevent slippage of said rod (16) in said grooves (22) of said clamp members (18).

12. The transpedicular vertebral attachment system as claimed in any of the claims 1 to 7, further comprising
- a cylindrical circular clamp member (29) open at one of its sides and housing therein a ball joint (31) which is also open at one side thereof;
- a pair of hollow cylinders (30) one of them each being attached to said open sides of said cylindrical circular clamp member (29), said hollow cylinders being designed to receive said screw member (1, 3, 34) before the latter is inserted into the bore of the plug member (2, 24); and
- a cylindrical rod (32) being designed to be inserted through said circular clamp member (29) and said ball joint (31), said rod (32) fitting to and being fixed by said clamp member (29) and said ball joint (31).

13. The transpedicular vertebral attachment system as claimed in claim 12, wherein the outer surfaces of said cylindrical rod (32) and/or the inner surfaces of said cylindrical circular clamp member (29) and of said ball joint (31) are diamond-point finished to prevent slippage of said rod (32) along said inner surfaces.

14. The transpedicular vertebral attachment system as claimed in claim 12 or in claim 13, wherein said screw member (1, 3, 34), once being inserted in said pair of hollow cylinders (30), is blocked therein by means of nuts (36).

## Patentansprüche

1. Transpedikulares Wirbel-Befestigungssystem, umfassend
- ein Dübelelement (2) mit einer Bohrung und einer Mehrzahl von sich in Längsrichtung erstreckenden Schlitzen (7);
- ein Schraubenelement (1, 3), das in die Bohrung des Dübelelements (2) eingesetzt ist, welches hierdurch in radialer Richtung ausgedehnt wird;
dadurch gekennzeichnet, daß das Dübelelement (2) einen runden Querschnitt und ein Gewinde (6) auf seiner Außenfläche und eine Einrichtung (5) zum Drehen des Dübelelements (2) umfaßt.

2. Transpedikulares Wirbel-Befestigungssystem nach Anspruch 1, worin die Einrichtung (5) zum Drehen des Dübelelements (2) eine hexagonale äußere Form aufweist, wodurch ein Eingreifen in einen hohlen Schraubendreher zur Handhabung möglich wird.

3. Transpedikulares Wirbel-Befestigungssystem nach Anspruch 1 oder Anspruch 2, worin das Dübelelement (2) aus einem Material hergestellt ist, das gewählt ist aus der Gruppe, die besteht aus Siliconkautschuk, einem anderen Polymer mit ähnlichen mechanischen Merkmalen und mit Bioverträglichkeit, Metall und Metall-Legierung.

4. Transpedikulares Wirbel-Befestigungssystem nach einem der Ansprüche 1 bis 3, worin das Schraubenelement (1, 3) die Form eines zylindrischen Stabes mit einem mit einem Gewinde versehenen Abschnitt (3) und einem in radialer Richtung aufgeweiteten Abschnitt (4) hat, welcher als Halt dient, der das Einführen des Schraubenelements (1, 3) in das Dübelelement (2) beschränkt.

5. Transpedikulares Wirbel-Befestigungssystem nach Anspruch 4, worin sich der mit einem Gewinde versehene Abschnitt (3) des Schraubenelements (1, 3) von der Länge her zwischen der Schraubenspitze und dem in radialer Richtung aufgeweiteten Abschnitt (4) erstreckt.

6. Transpedikulares Wirbel-Befestigungssystem nach Anspruch 5, worin der mit einem Gewinde versehene Abschnitt geringfügig länger ist als die Länge eines Pedikels, an den er angebracht werden soll.

7. Transpedikulares Wirbel-Befestigungssystem nach einem der Ansprüche 1 bis 6, worin das Schraubenelement (1, 3) hergestellt ist aus einem Material, das gewählt ist aus der Gruppe, die besteht aus Stahl, Titan oder einer Legierung.

8. Transpedikulares Wirbel-Befestigungssystem nach einem der Ansprüche 1 bis 7, weiter umfassend
- wenigstens zwei Paare Klemmelemente (18), wobei jedes Paar aus zwei einzelnen Klemmelementen besteht, die zwei Löcher (20, 21) und eine Rille (22) umfassen, wobei das erste der beiden Löcher (20) einen Innendurchmesser aufweist, der dem Außendurchmesser des Dübelelements (2, 24) entspricht und geeignet ist, das Gewinde (6) auf der Außenfläche des Dübelelements (2, 24) aufzunehmen, und wobei das zweite der beiden Löcher (21) einen geringeren Durchmesser als das erste Loch (20) aufweist und geeignet ist, eine Kleine Schraube (25) zum engen Zusammenhalten und Verbinden des Paars Klemmelemente (18) aufzunehmen und wobei die Rille (22) die Form einer Zylinderhälfte aufweist;
- ein Paar Rundkopf-Blindmuttern (26, 27), die auf jedes Dübelelement (2, 24) aufgeschraubt sind, die in den Löchern (20) des Paars Klemmelemente (18) untergebracht sind, wobei die Rundkopf-Blindmuttern (26, 27) das Paar Klemmelemente (18) fest zusammenhalten und verbinden können; und
- einen zylindrischen Stab (16), der einen Außendurchmesser aufweist, der dem Innendurchmesser des Paars Rillen (22) des Paars Klemmelemente (18) entspricht, wobei der Stab (16) in das Paar Rillen (22) paßt und von diesen fixiert wird.

9. Transpedikulares Wirbel-Befestigungssystem nach Anspruch 8, worin die beiden Klemmelemente (18) des Paars Klemmelemente (18) spiegelsymmetrisch in Bezug auf die aneinander fixierten Oberflächen sind.

10. Transpedikulares Wirbel-Befestigungssystem nach Anspruch 8 oder Anspruch 9, worin die Löcher (20) in den Klemmelementen (18) kugelförmig ausgehöhlte Abschnitte (28) zur Aufnahme des Paars Rundkopf-Blindmuttern (26, 27) aufweisen, die auf das Dübelelement (2, 24) aufgeschraubt sind.

11. Transpedikulares Wirbel-Befestigungssystem nach einem der Ansprüche 8 bis 10, worin die Außenflächen des zylindrischen Stabes (16) und/oder die Innenflächen (23) der Rillen (22) mit einer Diamat-Pointierung bearbeitet sind, um ein Rutschen des Stabes (16) in den Rillen (22) der Klemmelemente (18) zu verhindern.

12. Transpedikulares Wirbel-Befestigungssystem nach einem der Ansprüche 1 bis 7, weiter umfassend
- ein zylinderförmig rundes Klemmelement (29), das an einer seiner Seiten offen ist und darin ein Kugelgelenk (31) aufnimmt, das ebenfalls an einer seiner Seiten offen ist;
- ein Paar Hohlzylinder (30), von denen jeweils einer an den offenen Seiten des zylindrisch runden Klemmelements (29) befestigt ist, wobei die Hohlzylinder dafür vorgesehen sind, das Schraubenelement (1, 3, 34) aufzunehmen, bevor dieses in die Bohrung des Dübelelements (2, 24) eingesetzt wird; und
- einen zylindrischen Stab (32), der dafür vorgesehen ist, durch das runde Klemmelement (29) und das Kugelgelenk (31) hindurchgeführt zu werden, wobei der Stab (32) in das Klemmelement (29) und das Kugelgelenk (31) paßt und von diesen festgehalten wird.

13. Transpedikulares Wirbel-Befestigungssystem nach Anspruch 12, worin die Außenflächen des zylindrischen Stabes (32) und/oder die Innenflächen des zylinderförmig runden Klemmelements (29) und des Kugelgelenks (31) mit einer Diamant-Pointierung bearbeitet sind, um ein Rutschen des Stabes (32) entlang der genannten Innenflächen zu verhindern.

14. Transpedikulares Wirbel-Befestigungssystem nach Anspruch 12 oder Anspruch 13, worin das Schraubenelement (1, 3, 34), nachdem es einmal in das Paar Hohlzylinder (30) eingesetzt wurde, darin mittels Muttern (36) blockiert wird.

## Revendications

1. Système transpédiculé de fixation de vertèbres comportant
- un élément (2) formant cheville ayant un trou et une pluralité de fentes (7) s'étendant longitudinalement;
- un élément (1, 3) formant vis inséré dans le trou de l'élément (2) formant cheville qui s'y étend radialement;
caractérisé en ce que l'élément (2) formant cheville a une section transversale circulaire et comporte un filetage (6) sur sa surface extérieure, et des moyens (5) destinés à faire tourner l'élément (2) formant cheville.

2. Système transpédiculé de fixation de vertèbres suivant la revendication 1, dans lequel les moyens (5) destinés à faire tourner l'élément (2) formant cheville a une forme extérieure hexagonale qui permet la coopération avec un tournevis creux pour la manipulation.

3. Système transpédiculé de fixation de vertèbres suivant la revendication 1 ou la revendication 2, dans lequel l'élément (2) formant cheville est en un matériau choisi parmi le groupe comprenant le silastique, tout autre polymère ayant une biocompatibilité et des caractéristiques mécaniques similaires, en métal et en alliage de métal.

4. Système transpédiculé de fixation de vertèbres suivant l'une quelconque des revendications 1 à 3, dans lequel l'élément (1, 3) en forme de vis a la forme d'une tige cylindrique ayant une partie (3) filetée et une partie (4) élargie radialement agissant en tant que butée pour limiter l'insertion de l'élément (1, 3) formant vis dans l'élément (2) formant cheville.

5. Système transpédiculé de fixation de vertèbres suivant la revendication 4, dans lequel la partie (3) filetée de l'élément (1, 3) formant vis s'étend en longueur entre la pointe de vis et la partie (4) élargie radialement.

6. Système transpédiculé de fixation de vertèbres suivant la revendication 5, dans lequel la partie filetée est légèrement plus longue que la longueur d'un pédicule auquel on l'applique.

7. Système transpédiculé de fixation de vertèbres suivant l'une quelconque des revendications 1 à 6, dans lequel l'élément (1, 3) formant vis est en un matériau choisi parmi le groupe comprenant l'acier, le titanium ou un alliage.

8. Système transpédiculé de fixation de vertèbres suivant l'une quelconque des revendications 1 à 7, comportant en outre
- au moins deux paires d'éléments (18) formant pince, chaque paire consistant en deux éléments formant pince simples comportant deux trous (20, 21) et une rainure (22), le premier des deux trous (20) ayant un diamètre intérieur correspondant au diamètre extérieur de l'élément (2, 24) formant cheville et étant approprié pour recevoir le filetage (6) sur la surface extérieure de l'élément (2, 24) formant cheville, le second des deux trous (21) ayant un diamètre inférieur à celui du premier trou (20) et étant approprié pour loger une petite vis (25) pour maintenir et joindre les deux paires d'éléments (18) formant pince ensemble de manière étanche, et la rainure (22) ayant la forme d'une moitié d'un cylindre;
- une paire d'écrous aveugles (26, 27) à tête ronde vissés sur chacun des éléments (2, 24) formant cheville logé dans les trous (20) de la paire d'éléments (18) formant pince, ces écrous (26, 27) aveugles à tête ronde étant adaptés pour maintenir et joindre ensemble de manière étanche la paire d'éléments (18) formant pince; et
- une tige (16) cylindrique ayant un diamètre extérieur correspondant au diamètre intérieur de la paire de rainures (22) de la paire d'éléments (18) formant pince, cette tige (16) s'adaptant à la paire de rainures (22) et étant fixée par cette paire.

9. Système transpédiculé de fixation de vertèbres suivant la revendication 8, dans laquelle deux éléments (18) formant pince de la paire d'éléments (18) formant pince sont symétriques comme dans un miroir en ce qui concerne leurs surfaces par lesquelles ils sont fixés l'un à l'autre.

10. Système transpédiculé de fixation de vertèbres suivant la revendication 8 ou la revendication 9, dans lequel les trous (20) dans les éléments (18) formant pince ont des parties (28) qui ont été creusées de manière sphérique pour recevoir la paire d'écrous (26, 27) aveugles à tête ronde vissés sur l'élément (2, 24) formant cheville.

11. Système transpédiculé de fixation de vertèbres suivant l'une quelconque des revendications 8 à 10, dans lequel les surfaces extérieures des tiges (16) cylindriques et/ou les surfaces (23) intérieures des rainures (22) sont moletées pour empêcher le dérapage de la tige (16) dans les rainures (22) des éléments (18) formant pince.

12. Système transpédiculé de fixation de vertèbres suivant l'une quelconque des revendications 1 à 7, comportant en outre
- un élément (29) formant pince circulaire cylindrique ouvert à l'un de ses côtés et logeant un joint (31) à rotule qui est également ouvert à l'un de ses côtés;
- une paire de cylindres (30) creux qui sont chacun fixés au côté ouvert de l'élément (29) formant pince circulaire cylindrique, ces cylindres creux étant conçus pour recevoir l'élément (1, 3, 34) formant vis avant que ce dernier ne soit inséré dans le trou de l'élément (2,24) formant cheville; et
- une tige (32) cylindrique étant conçue pour être insérée par l'élément (29) formant pince circulaire et le joint (31) à rotule, cette tige (32) s'ajustant à l'élément (29) formant pince et au joint (31) à rotule et étant fixée par ceux-ci.

13. Système transpédiculé de fixation de vertèbres suivant la revendication 12, dans lequel les surfaces extérieures de la tige (32) cylindrique et/ou les surfaces intérieures de l'élément (29) formant pince circulaire cylindrique et les surfaces intérieures du joint (31) à rotule sont moletées pour empêcher le dérapage de la tige (32) le long des surfaces intérieures.

14. Système transpédiculé de fixation de vertèbres suivant la revendication 12 ou la revendication 13, dans lequel l'élément (1, 3, 34) formant vis, une fois qu'il est inséré dans la paire de cylindres (30) creux, y est bloqué au moyen d'écrous (36).
